# EUROPÄISCHE PATENTANMELDUNG

(11) **EP 2 286 786 A1**
(43) Veröffentlichungstag der Anmeldung: **23.02.2011**
(21) Anmeldenummer: 09167991.0
(22) Anmeldetag: 17.08.2009
(51) Int. Cl.: A61K 8/21, A61K 8/24, A61K 8/34, A61Q 11/00

(54) **Remineralisierendes Zahnpflegemittel**

(71) Anmelder: Weckerle Cosmetics Eislingen GmbH, 73054 Eislingen (DE)
(72) Erfinder: Meilick, Maren, 73728 Esslingen (DE); Casagranda, Birgitta, 70372 Stuttgart (DE)
(74) Vertreter: Hohgardt, Martin

(57) **Zusammenfassung**

Zahnpflegemittel zur Remineralisierung von demineralisierten Zähnen, aufweisend einen Wirkstoffkomplex aus Hydroxylapatit und Fluorid, wobei der Wirkstoffkomplex außerdem Xylitol zur Erhöhung der Remineralisierungswirkung durch das Hydroxylapatit und durch das Fluorid aufweist.

## Beschreibung

Die Erfindung betrifft ein Zahnpflegemittel zur Remineralisierung von demineralisierten Zähnen beziehungsweise Zahnhälsen.

Im Mund herrscht normalerweise ein Gleichgewicht zwischen Hydroxylapatit, welches sich aus dem Zahnschmelz löst und dem Hydroxylapatit, welches in den Zähnen durch chemische Wechselwirkungen mit Bestandteilen des Speichels neu gebildet wird. Dieses Gleichgewicht kann durch physikalische oder chemische Einflüsse gestört werden. So können beispielsweise Säuren aus Mundbakterien und Nahrungsmitteln das Hydroxylapatit angreifen und zersetzen. Wenn das Gleichgewicht gestört ist, kann es zu einer Demineralisierung des Zahnschmelzes und damit im schlimmsten Fall zum Zahnschmelzverlust kommen. Bei einer Rückbildung des Zahnschmelzes, wird weiches Dentin freigelegt und über Dentin-Kanälchen werden dann mechanische, chemische oder thermische Reize als Schmerzempfindung an die Zahnpulpa weitergegeben. Auch bildet der Zahnschmelz eine natürliche Barriere gegen Bakterien, ist diese nicht mehr intakt, so können Bakterien das darunter liegende Dentin angreifen und können in den Organismus eindringen und somit die allgemeine Gesundheit angreifen.

Natürliche Stoffe können diesen Rückbildungsprozess des Zahnschmelzes verlangsamen beziehungsweise durch Remineralisierung umkehren. Zum Beispiel hat Natriumfluorid eine remineralisierende Wirkung auf den Zahnschmelz. Außerdem ist die Verwendung von Hydroxylapatit zur Remineralisierung von Zahnstrukturen bekannt. Die derart bereitgestellte Remineralisierung ist allerdings häufig nicht ausreichend und findet lediglich an der Oberfläche der Zähne beziehungsweise der Zahnstrukturen statt.

Die Aufgabe der vorliegenden Erfindung besteht daher darin, ein Zahnpflegemittel bereit zu stellen, das eine stärkere Remineralisierung ermöglicht, bei der auch in tieferen Schichten des Zahnschmelzes eine Remineralisierung stattfindet.

Diese Aufgabe wird erfindungsgemäß durch ein Zahnpflegemittel gelöst, welches einen Wirkstoffkomplex aus Hydroxylapatit und Fluorid aufweist. Erfindungsgemäß weist der Wirkstoffkomplex außerdem Xylitol auf. Das Hinzufügen von Xylitol zusätzlich zum Hydroxylapatit und Fluorid Wirkstoffkomplex führt zu einer Erhöhung der Remineralisierungswirkung von Hydroxylapatit und Fluorid.

Mit Hilfe des erfindungsgemäßen Zahnpflegemittels wird eine höhere Remineralisierung erzielt und diese Remineralisierung geht bis in tiefere Schichten des Zahnschmelzes als dieses bei den im Stand der Technik bekannten Mittel zum Remineralisieren der Fall ist.

In einer bevorzugten Ausführungsform hat das in dem Zahnpflegemittel verwendete feinteilige Hydroxylapatit einen mittleren Durchmesser von 0,1 bis 10 µm. Vorzugweise befindet sich in dem Zahnpflegemittel das Hydroxylapatit bei Herstellung in einer Konzentration von etwa 1 bis 40 Gewichtsprozent, wobei in einer weiter bevorzugten Ausführungsform 15 bis 30 Gewichtsprozent verwendet werden. Während der Herstellung des Zahnpflegemittels wird dabei das Hydroxylapatit mit einem Kalziumfänger behandelt, so dass die Konzentration des Fluorids während der Lagerung nicht beeinflusst wird. Dabei hat das Fluorid vorzugsweise bei Herstellung einen Anteil von etwa 0,05 bis 0,15 Gewichtsprozent, vorzugsweise 0,1 bis 0,15 Gewichtsprozent an dem Zahnpflegemittel. Das im Zahnpflegemittel verwendete Fluorid kann dabei Zinn(II)-Fluorid, Aminfluorid, Natrium-Monofluorphosphat, Natriumfluorid oder einer Mischung dieser Fluoride enthalten. Grundsätzlich können Stoffe verwendet werden, die eine Fluorid Ionenquelle darstellen. Das im Zahnpflegemittel verwendete Xylitol hat vorzugweise einen Anteil von etwa 5 bis 20 Gewichtsprozent, vorzugsweise 10 bis 20 Gewichtsprozent.

Das erfindungsgemäße Zahnpflegemittel bestehend aus Fluorid, Hydroxylapatit und Xylitol und kann in Form aller für die Mund- und Zahnpflege geeigneten Präparate, wie Zahnpasten, Mundwässern, Mundwässerkonzentraten, Kaugummis und Kautabletten vorliegen.

In einer bevorzugten Ausführungsform ist das Zahnpflegemittel als Wirkstoffkomplex einer Zahnpaste beziehungsweise eines Gels ausgelegt. Dabei können dem Komplex bestehend aus Hydroxylapatit, Fluorid und Xylitol weitere Komponenten beigemengt werden, die in Zahnpasten und Gelen üblich sind. Hierbei kann es sich beispielsweise um Schleifmittel wie Kalkspat, Aluminiumhydroxid, kalziniertes Aluminiumoxid, Kalziumkarbonat, Natriumbikarbonat, Kalziumhydrogenphosphat oder Siliziumdioxid Hydrat handeln, die alleine oder in Kombination einen Gewichtsanteil von 2 bis 20 Prozent der Zahnpaste beziehungsweise des Gels ausmachen können.

Darüber hinaus können das Zahnpflegemittel als Feuchthaltemittel beispielsweise Glyzerin, Propylenglykol, D-Glucitol oder eine Mischung davon mit einem Gewichtsanteil von 10 bis 45 Prozent, vorzugsweise 25 bis 45 Prozent enthalten.

In der bevorzugten Ausführungsform des Zahnpflegemittels als Zahnpaste oder Gel können auch noch Verdickungsmittel beigemengt werden, wobei diese aus der Gruppe der Silikatverdickungsmittel, der Carboxymethyl-Zellulosen, der Natrium-Carboxymethyl-Zellulosen, der Polycarboxymethyl-Zelluloseether, des Xanthan-Gummis, der Celluloseglykolether, der Cellulose 2-Hydroxypropyl-Ether, der Alginsäuren oder der Johannisbrotkernmehle stammen können und bevorzugt einen Gewichtsanteil von 0,2 bis 3,0 Prozent ausmachen.

Des Weiteren können beispielsweise grenzflächenaktive Substanzen zur Schaumbildung der bevorzugten Ausführungsform beigemischt werden, wobei diese Substanzen hydrophobe und hydrophile chemische Gruppen aufweisen und als Tenside bezeichnet werden. Dabei können die grenzflächenaktiven Substanzen beispielsweise anionische wasserlösliche Salze der höheren Alkylsulfate oder Sulfoacetate sein, wie zum Beispiel Natriumlaurylsulfat, Natriumlaurylsulfoacetat oder andere passende Alkylsulfate oder Sulfoacetate mit 8 bis 18 Kohlenstoffatomen in der Alkylgruppe. Des Weiteren können auch wasserlösliche Salze von sulfonierten Monoglyzeriden von höheren Fettsäuren, wie zum Beispiel Kokosnuss-Monoglyzerid-Sulfonat oder andere geeignete sulfonierte Monoglyzeride von Fettsäuren mit 10 bis 18 Kohlenstoffatomen verwendet werden. Als weiterer Zusatz kann auch Natriumlaurylsulfatsalz von Amiden von höheren Fettsäuren, zum Beispiel Säuren mit 12 bis 16 Kohlenstoffatomen mit niedrigen aliphatischen Aminosäuren, wie zum Beispiel, Taurin oder Sarkosin dem Zahnpflegemittel beigefügt werden oder andere Aminosäuren mit 2 bis 6 Kohlenstoffatomen, wie zum Beispiel Natrium-N-methyl-N-palmitoyl Taurid, Natrium N-lauroyl-, N-myristoyl- und N-palmitoyl Sarkosinate. Auch lassen sich wasserlösliche Salze von Estern von solchen Fettsäuren mit isethionischen Säuren oder Glyzerinmonosulfate verwenden, wie zum Beispiel Kokosfettsäure-Isethionat und das Natriumsalz des monosulfanierten Monoglyzerins von hydrierten Kokosnussfettsäuren. Es können aber auch wasserlösliche Salze von Olefinsulfonaten, zum Beispiel Natrium C14-16 Olefinsulfonat, Alkensulfonate oder Hydroxyalkensulfonate oder eine Mischung daraus verwendet werden, wenn diese mindestens 12 bis 16 Kohlenstoffatome aufweisen. Es können aber auch Seifen von höheren Fettsäuren verwendet werden, zum Beispiel welche die 12 bis 18 Kohlenstoffatome aufweisen, wie beispielsweise Kokosnussfettsäure. Auch amphotere grenzflächenaktive Substanzen wie zum Beispiel Betaine können verwendet werden. Die grenzflächenaktive Substanz macht dabei vorzugsweise einen Gewichtsanteil von 1 bis 5 Prozent aus.

Des Weiteren kann die bevorzugte Ausführungsform des Zahnpflegemittels als Zahnpaste auch Geschmacksmittel, Süßstoffe und farbgebende Subtanzen enthalten. Als Geschmacksmittel können beispielsweise Öle eingesetzt werden, beispielsweise Minzöl, Pfefferminzöl, Wintergrünöl, Nelkenöl, Salbeiöl, Salcicylsäuremethylester, Anethole oder eine Mischung aus diesen. Beispiele für Süßstoffe beinhalten beispielsweise Laktose, Maltose, Mononatriumsalz und Saccharine. Die Mischung der Geschmacksmittel und Süßstoffe machen dabei bevorzugt einen Gewichtsanteil von 0,1 bis 5 Prozent aus. Vorzugsweise liegt der Gewichtsanteil des Geschmacksmittels bei 0,5 bis 2 Prozent und des Süßstoffes bei 0,1 bis 4 Prozent, wobei bei künstlichen Süßstoffen wie zum Beispiel Saccharin ein Anteil von 0,1 bis 0,5 ausreicht.

Des Weiteren kann die bevorzugte Ausführungsform des Zahnpflegemittels als Zahnpaste auch Titandioxid und andere farbgebende Pigmente aufweisen, die vorzugsweise in einem Anteil von 0,05 bis 2 Prozent enthalten sind.

Die Erfindung wird durch die folgenden Beispiele näher erläutert, ist jedoch nicht auf dieselben beschränkt.

### Beispiel 1

| **Inhaltsstoff** | **Gewichtsprozent** |
|---|---|
| Hydroxylapatit (mittlere Korngröße 0,1 - 10 µm) | 30,000 |
| Wasser | 28,250 |
| Sorbitol | 10,500 |
| Xylitol | 10,000 |
| hydriertes Siliziumdioxid | 6,250 |
| Propylenglykol | 5,000 |
| tetrapotassium Pyrophosphat | 5,000 |
| Natrium C14-16 Olefin Sulfonat | 1,700 |
| CI 77891 / Farbstoff | 1,000 |
| Aroma | 1,000 |
| Carboxymethylcellulose | 0,800 |
| Natriumfluorid | 0,320 |
| Natriumsaccharin | 0,180 |
| **Summe** | **100,00** |

### Beispiel 2

| **Inhaltsstoff** | **Gewichtsprozent** |
|---|---|
| Wasser | 31,380 |
| Hydroxylapatit (mittlere Korngröße 0,1 - 10 µm) | 15,000 |
| Xylitol | 15,000 |
| hydriertes Siliziumdioxid | 12,500 |
| Sorbitol | 10,000 |
| Propylenglykol | 9,000 |
| tetrapotassium Pyrophosphat | 2,500 |
| Natrium C14-16 Olefin Sulfonat | 1,500 |
| Aroma | 1,000 |
| CI 77891 / Farbstoff | 0,800 |
| Xanthangummi | 0,800 |
| Natriumfluorid | 0,320 |
| Natriumsaccharin | 0,200 |
| **Summe** | **100,00** |

### Beispiel 3

| **Inhaltsstoff** | **Gewichtsprozent** |
|---|---|
| Wasser | 31,417 |
| Hydroxylapatit (mittlere Korngröße 0,1 - 10 µm) | 20,000 |
| hydriertes Siliziumdioxid | 11,250 |
| Sorbitol | 10,000 |
| Xylitol | 10,000 |
| Propylenglykol | 9,000 |
| tetrapotassium Pyrophosphat | 3,333 |
| Natrium C14-16 Olefin Sulfonat | 1,700 |
| CI 77891 / Farbstoff | 1,000 |
| Aroma | 1,000 |
| Carboxymethylcellulose | 0,800 |
| Natriumfluorid | 0,320 |
| Natriumsaccharin | 0,180 |
| **Summe** | **100,00** |

## Patentansprüche

1. Zahnpflegemittel zur Remineralisierung von demineralisierten Zähnen, aufweisend einen Wirkstoffkomplex aus Hydroxylapatit und Fluorid
**dadurch gekennzeichnet, dass**
der Wirkstoffkomplex außerdem Xylitol zur Erhöhung der Remineralisierungswirkung durch das Hydroxylapatit und durch das Fluorid aufweist.

2. Zahnpflegemittel gemäß Anspruch 1, **dadurch gekennzeichnet, dass** das Mittel das Hydroxylapatit in einer Konzentration von 1 bis 40 Gewichtsprozent, vorzugsweise 15 bis 30 Gewichtsprozent, enthält.

3. Zahnpflegemittel nach einem der vorherigen Ansprüche, **dadurch gekennzeichnet, dass** das Hydroxylapatit einen mittleren Durchmesser von etwa 0,1 µm bis 10 µm aufweist.

4. Zahnpflegemittel nach einem der vorherigen Ansprüche, **dadurch gekennzeichnet, dass** es sich bei dem Fluorid um Zinn(II)-Fluorid, Aminfluorid, Natrium-Monofluorphosphat und/oder Natriumfluorid handelt.

5. Zahnpflegemittel nach einem der vorherigen Ansprüche, **dadurch gekennzeichnet, dass** das Mittel das Fluorid in einer Konzentration von 0,05 bis 0,15 Gewichtsprozent, vorzugsweise 0,1 bis 0,15 Gewichtsprozent, enthält.

6. Zahnpflegemittel nach einem der vorherigen Ansprüche, **dadurch gekennzeichnet, dass** das Mittel das Xylitol in einer Konzentration von 5 bis 30 Gewichtsprozent, vorzugsweise 10 bis 20 Gewichtsprozent, enthält.

7. Zahnpflegemittel nach einem der vorherigen Ansprüche, **dadurch gekennzeichnet, dass** das Mittel ein Feuchthaltemittel aufweist, wobei es sich bei dem Feuchthaltemittel um Glyzerin, D-Glucitol, und/oder Propylenglykol handelt.

8. Zahnpflegemittel nach einem der vorherigen Ansprüche, **dadurch gekennzeichnet, dass** das Mittel einen Binder, ein Geliermittel und/oder ein Verdickungsmittel aufweist.

9. Zahnpflegemittel nach einem der vorherigen Ansprüche, **dadurch gekennzeichnet, dass** das Mittel eine grenzflächenaktive Substanz zur Schaumbildung aufweist, wobei die Substanz hydrophobe und hydrophile chemische Gruppen enthält.

10. Zahnpflegemittel nach einem der Ansprüche 1 bis 9, **dadurch gekennzeichnet, dass** das Mittel Bestandteil einer Zahnpasta ist.

11. Zahnpflegemittel nach einem der Ansprüche 1 bis 9, **dadurch gekennzeichnet, dass** das Mittel Bestandteil eines Mundwassers ist.

12. Zahnpflegemittel nach einem der Ansprüche 1 bis 9, **dadurch gekennzeichnet, dass** das Mittel Bestandteil eines Kaugummis ist.

13. Zahnpflegemittel nach einem der Ansprüche 1 bis 9, **dadurch gekennzeichnet, dass** das Mittel Bestandteil einer Kautablette ist.
